# EUROPÄISCHE PATENTSCHRIFT

(11) **EP 1 626 763 B1**
(45) Veröffentlichungstag und Bekanntmachung des Hinweises auf die Patenterteilung: **21.11.2007**
(21) Anmeldenummer: 03781992.7
(22) Anmeldetag: 19.12.2003
(51) Int. Cl.: A61M 16/00

(54) **MEDIENVERSORGUNGSSYSTEM**
MEDIA-SUPPLY SYSTEM
SYSTEME DE DELIVRANCE DE SUBSTANCES

(30) Priorität: 05.03.2003 AT 3272003
(43) Veröffentlichungstag der Anmeldung: 22.02.2006
(73) Patentinhaber: Kaltenegger, Andreas, 5020 Salzburg (AT); Waldhör, Peter, 5020 Salzburg (AT)
(72) Erfinder: Kaltenegger, Andreas, 5020 Salzburg (AT); Waldhör, Peter, 5020 Salzburg (AT)
(74) Vertreter: Gibler, Ferdinand
(86) Internationale Anmeldenummer: PCT/AT2003/000378
(87) Internationale Veröffentlichungsnummer: WO 2004/078245

(56) Entgegenhaltungen:
- GB-A- 933 172
- GB-A- 1 198 775
- US-A- 5 980 289
- US-B1- 6 346 014

## Beschreibung

Die Erfindung betrifft ein Medienversorgungssystem für medizinische Geräte mit einem ersten Verbindungsstück, welches zumindest eine erste Seite mit wenigstens zwei ersten Mediensteckern aufweist, wobei wenigstens ein zweites Verbindungsstück vorgesehen ist, welches den ersten Mediensteckem gegengleiche zweite Medienstecker aufweist, wobei die Verbindungen der ersten Medienstecker mit den korrespondierenden zweiten Mediensteckern mittels einer Bewegung des zweiten Verbindungsstücks gemeinsam herstellbar und/oder lösbar sind.

Bekannte solche Medienversorgungssysteme werden insbesondere im Bereich von Intensivstationen verwendet, wo eine Vielzahl medizinischer Geräte an einem Krankenbett angeordnet sind. Dabei sind an der Wand Anschlußmöglichkeiten für verschiedene Medien, wie z.B. Stromversorgung, Datenleitungen, Sauerstoffleitungen, Stickstoffleitungen od. dgl., vorgesehen, an die die einzelnen Geräte angeschlossen werden können. Ist eine Verlegung eines Patienten erforderlich, z.B. um eine Operation oder eine Untersuchung durchzuführen, so ist es erforderlich, die Medienversorgung der einzelnen Geräte abzuschließen und durch eine transportable Quelle, z.B. eine Batterie, ein Generator, Gasflaschen od. dgl., sicherzustellen. Nachteilig an diesen Systemen ist es, daß vor und nach einer Verlegung sämtliche Geräte einzeln neu angeschlossen werden müssen, wobei zumindest kurzfristig eine Betriebsunterbrechung der Geräte auftreten kann. Aufgrund der Vielzahl an Kabel und Schläuchen kann es bei einer Unachtsamkeit leicht dazu kommen, daß ein falsches Kabel bzw. ein falscher Schlauch unbeabsichtigt gelöst wird, wodurch es zu einer längeren Betriebsunterbrechung kommen kann. Weiters besteht bei einer Vielzahl an Kabel und Schläuchen die Gefahr, daß jemand über ein Kabel oder einen Schlauch stolpert und eine Verbindung versehentlich löst

Aus der US 6 346 014 B1 ist ein Stecker für medizinische Geräte bekannt, welcher zwei getrennte Kontakte aufweist. Mittels einer Translation des Steckers werden Verbindungen der Kontakte mit gegengleichen Anschlüssen hergestellt.

Die GB 1 198 775 A betrifft einen elektrischen Schalter für eine Vielzahl an elektrischen Schaltern, wobei die elektrischen Schalter mittels einer Schwenkbewegung geschlossen werden. Anstelle mit elektrischen Schaltern ist auch eine Ausbildung mit Fluid-Steckern vorgesehen.

Aufgabe der Erfindung ist es ein Medienversorgungssystem der eingangs genannten Art weiterzubilden, bei dem die bekannten Nachteile vermieden werden und das einen einfachen, schnellen und zuverlässigen Anschluß eines Gerätes an verschiedene Medien ermöglicht. Weitere Aufgabe der Erfindung ist es, ein Medienversorgungssystem anzugeben, bei dem auch bei einem Umschließen der Medienzuleitung eine weitgehend kontinuierliche Versorgung gewährleistet werden kann. Eine weitere Aufgabe der Erfindung ist es, ein Medienversorgungssystem anzugeben, bei dem der Anschluß an eine falsche Medienleitung und/oder das Vergessen eines Anschlusses weitgehend unterbunden werden.

Erfindungsgemäß wird dies dadurch erreicht, daß zumindest einer der ersten Medienstecker ein erster Stromstecker und zumindest einer der ersten Medienstecker ein erster Stecker für ein Fluid, insbesondere für ein Gas, ist, daß das zweite Verbindungsstück um eine Schwenkachse gegenüber dem ersten Verbindungsstück verschwenkbar ist, und dass der Abstand des ersten Stromsteckers von der Schwenkachse größer als der Abstand des zumindest einen ersten Steckers für ein Fluid von der Schwenkachse ist.

Dadurch ergibt sich der Vorteil, daß mehrere Medienleitungen in einem Arbeitsschritt verbunden bzw. gelöst werden können, wodurch die erforderlichen Zeiten für das Anschließen bzw. das Abschließen der Geräte erheblich reduziert werden können. Ein weiterer Vorteil ist, daß durch die Ausgestaltung der Verbindungsteile vorgegeben ist, welche Stecker miteinander verbunden werden, sodaß irrtümliche Anschlüsse an falschen Medienleitungen zuverlässig unterbunden werden. Weiters wird sichergestellt, daß alle Verbindungen hergestellt werden und nicht ein Medienanschluß vergessen werden kann. Mittels des erfindungsgemäßen Medienversorgungssystem kann die Versorgung mit unterschiedlichen Medien auf einfache Weise sichergestellt werden. In einem Arbeitsschritt können die Verbindungen unterschiedlicher Medien in einer zeitlich vorgebbaren Reihenfolge geschlossen und/oder geöffnet werden. Dabei kann z.B. ausgeschlossen werden, daß ein explosives Gas aus einem Medienstecker entweichen kann, während ein Stromstecker getrennt wird, wobei Funken auftreten können. Die Schwenkachse stellt weiters eine einfache und sichere Führung der Verbindungsstücke dar. Weiters kann sichergestellt werden, daß beim Öffnen der Verbindungen die Stromstecker vor den Steckern für Fluide geöffnet werden. Tritt bei dem Öffnen der Stromstecker ein Funken auf und tritt aus den Steckern für Fluide ein explosives Gas aus, so ist durch diese Ausbildung gewährleistet, daß das Gas erst austreten kann, nachdem der Funke wieder erloschen ist.

In Weiterbildung der Erfindung kann vorgesehen sein, daß die Schwenkachse im Wesentlichen normal zur Längsachse der ersten Medienstecker angeordnet ist.

In weiterer Ausbildung der Erfindung kann vorgesehen sein, daß das zweite Verbindungsstück an einem der Schwenkachse gegenüberliegenden Ende mittels einem Verriegelungselement lösbar mit dem ersten Verbindungsstück verbindbar ist. Dabei kann sichergestellt werden, daß alle Medienstecker ordnungsgemäß verbunden sind, sofern das zweite Verbindungsstück mit dem ersten Verbindungsstück verriegelt ist. Weiters kann aufgrund des Verriegelungselementes einfach festgestellt werden, ob alle Medienstecker ordnungsgemäß verbunden sind.

Gemäß einer anderen Ausgestaltung der Erfindung kann vorgesehen sein, daß das zweite Verbindungsstück mit dem ersten Verbindungsstück lösbar verbindbar ist, wobei die Schwenkachse durch die Verbindung ausbildbar ist. Dadurch kann das erste Verbindungsstück von dem zweiten Verbindungsstück vollständig getrennt werden, wodurch bei einer Verlegung z.B. das erste Verbindungsstück mit den zu transportierenden Geräten und das zweite Verbindungsstück mit einer stationären Medienquelle und/oder einer mobilen Medienquelle verbunden sein kann. Vor dem Transport muß lediglich das erste Verbindungsstück von der stationären Medienquelle getrennt und mit der mobilen Medienquelle verbunden werden. Dieser Vorgang ist einfach und schnell handhabbar, wobei insbesondere durch eine geeignete Führung ein schnelles An- und Abstecken sichergestellt werden kann.

In Weiterführung der Erfindung kann vorgesehen sein, daß wenigstens einer der ersten Medienstecker beweglich in dem ersten Verbindungsstück und/oder wenigstens einer der zweiten Medienstecker beweglich in dem zweiten Verbindungsstück gelagert ist. Dadurch kann die erforderliche Kraft zum Schließen und/oder Öffnen der Medienstecker gering gehalten werden, welche eine translatorische und/oder rotatorische Bewegung ausführen können.

Gemäß einer weiteren Ausführungsform der Erfindung kann vorgesehen sein, daß ein erster Kontaktstift zum Schließen eines Stromkreises beweglich an der ersten Seite des ersten Verbindungsstücks angeordnet ist. Dadurch kann sichergestellt werden, daß ein Stromkreis durch einen Taster od. dgl. und nicht durch das Lösen von Kontakten beim Trennen der Medienstecker getrennt wird, wodurch die Gefahr einer Funkenbildung verringert wird. Weiters wird sichergestellt, daß der erste Stromstecker im offenen Zustand des Medienversorgungssystems jedenfalls stromlos ist.

Gemäß einer anderen Ausbildung der Erfindung kann vorgesehen sein, daß ein zweiter Kontaktstift zum Schließen eines Stromkreises beweglich an dem zweiten Verbindungsstück angeordnet ist. Dadurch kann sichergestellt werden, daß ein Stromkreis durch einen Taster od. dgl. und nicht durch das Lösen von Kontakten beim Trennen der Medienstecker getrennt wird, wodurch die Gefahr einer Funkenbildung verringert wird. Weiters wird sichergestellt, daß der zweite Stromstecker im offenen Zustand des Medienversorgungssystems jedenfalls stromlos ist.

In Weiterbildung der Erfindung kann vorgesehen sein, daß der Stromstecker in einer Stromkammer und der Stecker für ein Fluid in einer von der Stromkammer getrennten Gaskammer angeordnet sind. Dadurch kann wirksam verhindert werden, daß ein in dem Medienversorgungssystem entstehender Funke mit einem explosiven Gas in Kontakt kommen kann.

Gemäß einer weiteren Ausgestaltung der Erfindung kann vorgesehen sein, daß die Gaskammer Belüftungsöffnungen aufweist, wodurch freiwerdende Gase aus dem Medienversorgungssystem sofort entweichen können und sich keine Speicher mit höheren Gas-Konzentrationen ausbilden.

Gemäß einer anderen Ausführungsform der Erfindung kann vorgesehen sein, daß das erste Verbindungsstück wenigstens zwei erste Stecker für ein Fluid aufweist, wobei diese ersten Stecker identisch ausgebildet sind. Durch die eindeutige Zuordnung der Stecker für ein Fluid durch die Anordnung im erfindungsgemäßen Medienversorgungssystem erscheint es nicht erforderlich zu sein, für unterschiedliche Fluide unterschiedliche Steckergeometrien vorzusehen, da eine Fehlbedienung weitgehend ausgeschlossen ist. Durch die identische Ausbildung kann insbesondere die Wartung und Instandhaltung vereinfacht werden, wobei insbesondere eine geringere Anzahl unterschiedlicher Ersatzteile erforderlich ist.

In weiterer Ausgestaltung der Erfindung kann vorgesehen sein, daß an dem ersten Verbindungsstück wenigstens eine zweite Seite mit dritten Mediensteckern für die Aufnahme eines weiteren zweiten Verbindungsstückes ausgebildet ist. Dadurch kann auf einfache Weise eine unterbrechungsfreie Umschaltung von stationären Medienquellen zu mobilen Medienquellen erfolgen, wobei kurzfristig beide Arten von Medienquellen mit dem ersten Verbindungsstück verbunden sein können.

In diesem Zusammenhang kann in Weiterbildung der Erfindung vorgesehen sein, daß an dem ersten Verbindungsstück Anschlüsse vorgesehen sind, die jeweils mit zumindest einem der ersten Medienstecker und einem der dritten Medienstecker verbunden sind. Dadurch können die Anschlüsse parallel über zwei zweite Verbindungsstücke versorgt werden, wodurch ein unterbrechungsfreies Umschalten auf einfache Weise vorgesehen sein kann.

Gemäß einer anderen Ausgestaltung der Erfindung kann vorgesehen sein, daß den ersten Steckern für Fluide und den dritten Steckern für Fluide jeweils ein Rückschlagventil zugeordnet ist. Durch das Rückschlagventil kann wirksam verhindert werden, daß ein Fluid von einem ersten Stecker für ein Fluid über einen dritten Stecker für ein Fluid strömt. Auch ein Fluidstrom in entgegengesetzter Richtung wird ausgeschlossen. Ein Fluid kann jeweils nur von einem zweiten Verbindungsstück über die Anschlüsse strömen, nicht jedoch in ein anderes zweites Verbindungsstück.

Gemäß einer weiteren Ausbildung der Erfindung kann vorgesehen sein, daß zwischen dem ersten Stromstecker und dem dritten Stromstecker ein Trenntrafo od. dgl. zwischengeschaltet ist. Dadurch kann sichergestellt werden, daß Strom nicht unmittelbar von einem zweiten Verbindungsstück in ein anderes zweites Verbindungsstück strömen kann.

In weiterer Ausgestaltung der Erfindung kann vorgesehen sein, daß eine Steuerung für ein im wesentlichen unterbrechungsfreies Umschalten einer Versorgung der Anschlüsse über die ersten Medienstecker zu einer Versorgung der Anschlüsse über die dritten Medienstecker vorgesehen ist. Dadurch kann der Umschaltvorgang bei zwei oder mehr angeschlossenen zweiten Verbindungsstücken an die jeweiligen Erfordernisse besonderes einfach und wirkungsvoll angepaßt werden.

Die Erfindung wird unter Bezugnahme auf die beigeschlossenen Zeichnungen, in welchen Ausführungsformen dargestellt sind, näher beschrieben. Dabei zeigt:
Fig. 1 eine Ausführungsform eines erfindungsgemäßen Medienversorgungssystems mit einem ersten Verbindungsstück und zwei zweiten Verbindungsstücken im Schrägriß;
Fig. 2 eine Explosionsseite einer ersten Seite des ersten Verbindungsstücks nach Fig. 1 mit ersten Mediensteckern;
Fig. 3 eine Explosionsdarstellung des ersten Verbindungsstücks nach Fig. 1;
Fig. 4 eine Explosionsdarstellung eines Verriegelungselementes des Medienversorgungssystems nach Fig. 1;
Fig. 5 eine Explosionsdarstellung des zweiten Verbindungsstücks nach Fig. 1; und
Fig. 6 eine Explosionsdarstellung eines Teils des zweiten Verbindungsstücks nach Fig. 5 mit zweiten Mediensteckem.

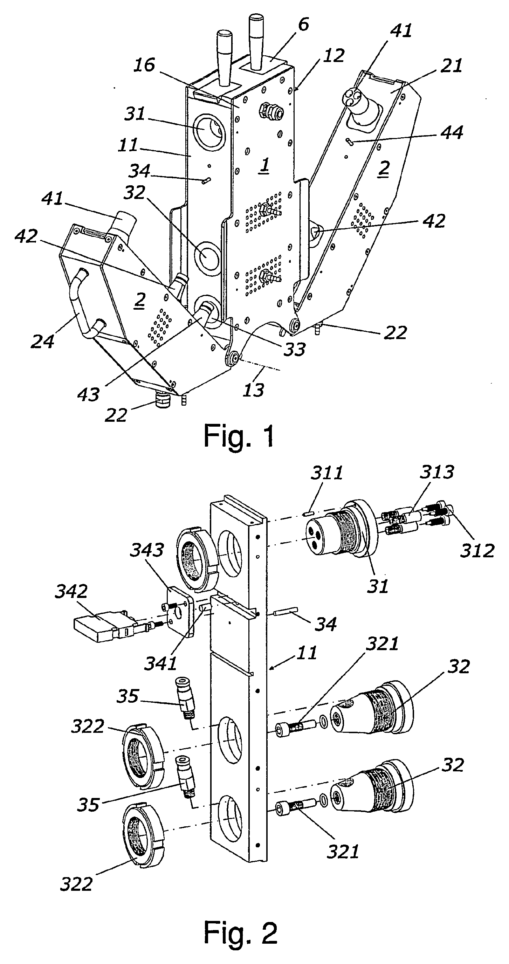

In Fig. 1 ist eine Ausführungsfonn eines erfindungsgemäßen Medienversorgungssystems für medizinische Geräte mit einem ersten Verbindungsstück 1, welches eine erste Seite 11 mit wenigstens zwei ersten Mediensteckern 31, 32, 33 aufweist, und einem zweiten Verbindungsstück 2 dargestellt, welches den ersten Mediensteckem 31, 32, 33 gegengleiche zweite Medienstecker 41, 42, 43 aufweist, wobei die Verbindungen der ersten Medienstecker 31, 32, 33 mit den korrespondierenden zweiten Mediensteckem 41, 42, 43 mittels einer Bewegung des zweiten Verbindungsstückes 2 gemeinsam herstellbar und/oder lösbar sind.

Dadurch können alle Verbindungen der ersten Medienstecker 31, 32, 33 und der zweiten Medienstecker 41, 42, 43 in einem Arbeitsschritt hergestellt und/oder gelöst werden, wobei sichergestellt ist, daß alle und nicht nur ein Teil der Verbindungen hergestellt werden. Dadurch kann eine zuverlässige Versorgung eines Geräts, insbesondere eines medizinischen Geräts, schnell und einfach sichergestellt werden.

Als Medien kommen insbesondere Gase, Flüssigkeiten, Energie, Daten od. dgl. in Betracht, wobei die Anzahl und Anordnung der Medienstecker je nach Bedarf variieren kann. Für eine Vielzahl an Anwendungen hat es sich als günstig erwiesen, wenn zumindest einer der ersten Medienstecker 31, 32, 33 ein erster Stromstecker 31 und zumindest einer der ersten Medienstecker 31, 32, 33 ein erster Stecker für ein Fluid 32, 33 ist, wobei das Fluid insbesondere ein Gas sein kann.

Aus Fig. 1 ist ersichtlich, daß das zweite Verbindungsstück 2 um eine Schwenkachse 13 gegenüber dem ersten Verbindungsstück 1 verschwenkbar ist. Das zweite Verbindungsstück 2 ist an einem der Schwenkachse 13 gegenüberliegenden Ende 21 mittels einem Verriegelungselement 6 lösbar mit dem ersten Verbindungsstück 1 verbindbar. Das Einrasten des Verriegelungselementes 6 kann einfach überprüft werden, wodurch eine einfache und sichere Kontrolle einer ordnungsgemäßen Verbindung zwischen dem ersten Verbindungsstück 1 und dem zweiten Verbindungsstück 2 gegeben ist. Weiters kann bereits bei der Herstellung der Verbindung das Zustandekommen der Verriegelung überprüft werden. Durch die Schwenkachse 13 ist eine einfache und zuverlässige Führung des zweiten Verbindungsstücks 2 gegeben, wodurch eine Fehlbedienung weitgehend ausgeschlossen werden kann.

Der Abstand des ersten Stromsteckers 31 von der Schwenkachse 13 kann größer als der Abstand des ersten Steckers für ein Fluid 32, 33 von der Schwenkachse 13 sein. Dadurch kann gewährleistet werden, daß das Lösen des ersten Stromsteckers 31 vor dem Lösen des ersten Steckers für ein Fluid 32, 33 erfolgt. Tritt dabei ein Funken auf, so ist der erste Stecker für ein Fluid 32, 33 noch verbunden, wodurch beim Lösen des ersten Steckers für ein Fluid 32, 33 gegebenenfalls austretende Gase erst nach dem Erlöschen des Funkens austreten können und durch den Funken nicht entzündet werden können.

Das Auftreten von Funken beim Lösen des ersten Stromsteckers 31 kann auch unterbunden werden, wenn ein erster Kontaktstift 34 zum Schließen eines Stromkreises beweglich an der ersten Seite 11 des ersten Verbindungsstücks 1 angeordnet ist. Durch den Kontaktstift 34 kann der Stromkreis bereits unterbrochen werden, wenn der erste Stromstecker 31 noch in Kontakt mit dem zweiten Stromstecker 41 ist. Es kann auch vorgesehen sein, daß ein zweiter Kontaktstift 44 zum Schließen eines Stromkreises beweglich an dem zweiten Verbindungsstück angeordnet ist.

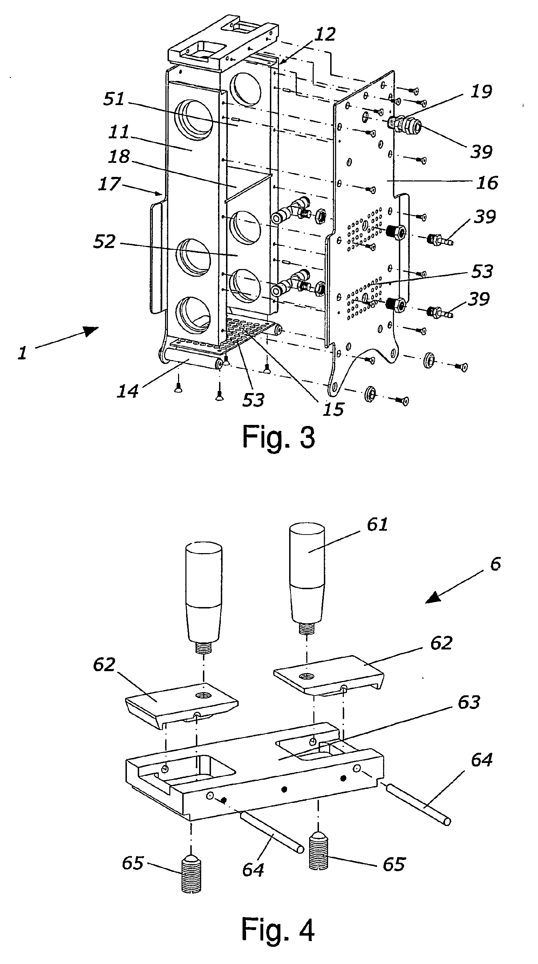

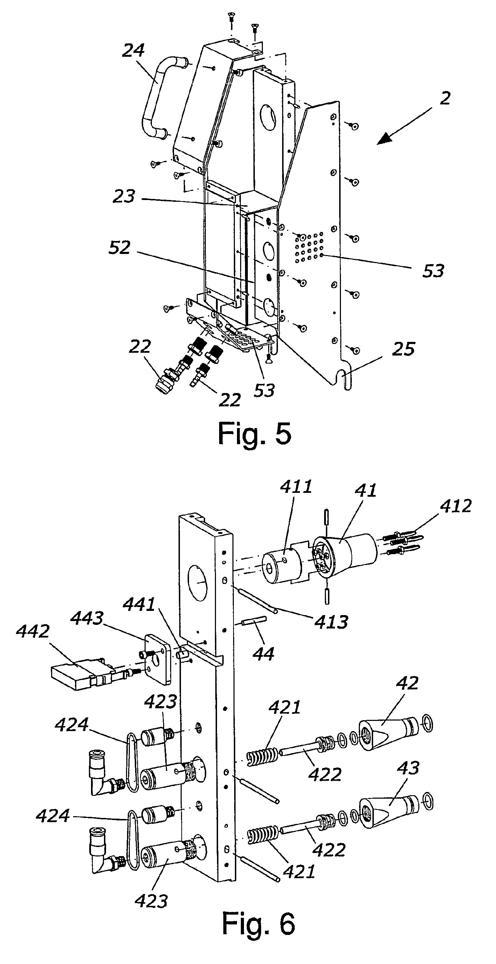

Ein von dem zweiten Verbindungsstück 2 unabhängiger Transport des ersten Verbindungsstücks 1 kann sichergestellt werden, wenn das zweite Verbindungsstück 2 lösbar mit dem ersten Verbindungsstück 1 verbindbar ist, wobei das erste Verbindungsstück 1 vollständig von dem zweiten Verbindungsstück 2 getrennt werden kann. Dabei kann bei der Verbindung die Schwenkachse 13 ausgebildet werden. Dazu kann das erste Verbindungsstück 1, wie in Fig. 3 gezeigt, einen Schwenkbolzen 14 aufweisen, wobei das zweite Verbindungsstück 2, wie aus Fig. 5 ersichtlich, eine Nut 25 aufweist, in die der Schwenkbolzen 14 unter Ausbildung der Schwenkachse 13 einbringbar ist.

Bei der in Fig. 1 gezeigten Ausführungsform können zwei zweite Verbindungsstücke 2 mit dem ersten Verbindungsstück 1 verbunden werden. In anderen Ausführungsformen kann auch nur ein zweites Verbindungsstück 2 oder mehr als zwei zweite Verbindungsstücke 2 vorgesehen sein. Können mindestens zwei zweite Verbindungsstücke 2 mit dem ersten Verbindungsstück 1 verbunden werden, so kann auf einfache Weise eine unterbrechungsfreie Versorgung eines Gerätes bei einer Umschaltung zwischen zwei Medienquellen sichergestellt werden, wobei zumindest kurzzeitig zwei zweite Verbindungsstücke 2 unterschiedlicher Medienquellen an dem ersten Verbindungsstück 1 angeschlossen sein können. Dazu weist das erste Verbindungsstück wenigstens eine zweite Seite 12 mit dritten Mediensteckern für die Aufnahme des weiteren zweiten Verbindungsstückes 2 auf. Die dritten Medienstecker können vorteilhafterweise gleich wie die ersten Medienstecker 31, 32, 33 ausgebildet sein.

In Fig. 2 ist eine Explosionszeichnung der ersten Medienstecker 31, 32, 33 und der ersten Seite 11 des ersten Verbindungsstückes 1 dargestellt. Der erste Stromstecker 31 weist einen Zentrierstift 311, Bananenbuchsen 312 und Buchsendistanzen 313 auf. Mit dem ersten Kontaktstift 34 ist ein Schaltstift 341 betätigbar, welcher mit einem an einer Halteplatte 343 befestigten Schaltelement 342 verbunden ist.

Bei der gezeigten Ausführungsform sind zwei ersten Stecker für ein Fluid 32, 33 vorgesehen, welche identisch ausgebildet sind. Da bereits durch die Anordnung eine Fehlbedienung weitgehend ausgeschlossen werden kann, ist es nicht erforderlich die ersten Stecker für ein Fluid 32, 33 unterschiedlich auszugestalten. Dadurch kann das erfindungsgemäße Medienversorgungssystem einfach ausgestaltet werden. Die ersten Stecker für ein Fluid umfassen jeweils eine Druckstiftschraube 321 und sind mit einer Sicherungsmutter 322 befestigt.

Den ersten Steckern für Fluide 32, 33 ist jeweils ein Rückschlagventil 35 zugeordnet. Dadurch kann sichergestellt werden, daß ein Fluid nicht über einen der ersten Stecker für Fluide 32, 33 aus dem ersten Verbindungsstück 1 strömen kann.

In Fig. 3 ist eine Explosionszeichnung des ersten Verbindungsstücks 1 dargestellt, welches einen Gehäuseboden 15, eine Vorderseite 16 und eine Rückseite 17 aufweist. Weiters ist ein Trennblech 18 vorgesehen, welches eine Stromkammer 51 von einer Gaskammer 52 abtrennt. Dabei ist der Stromstecker 31 in der Stromkammer 51 und der Stecker für ein Fluid 32, 33 in der von der Stromkammer 51 getrennten Gaskammer 52 angeordnet. Die Gaskammer 52 weist Belüftungsöffnungen 53 auf, die das rasche Entweichen eines gegebenenfalls austretenden Gases ermöglicht. Durch die Trennung der Stromkammer 51 von der Gaskammer 52 kann sichergestellt werden, daß austretende Gase nicht durch in der Stromkammer 51 auftretende Funken entzündet werden können. Weiters wird durch die Belüftungsöffnungen 53 erreicht, daß austretendes Gas nicht in dem ersten Verbindungsstück 1 gespeichert wird, wobei eine Konzentrationserhöhung auftritt. Gas könnte z.B. aufgrund einer Undichtheit in dem ersten Verbindungsstück 1 austreten.

An dem ersten Verbindungsstück 1 sind Anschlüsse 39 vorgesehen, welche eine Kabelverschraubung umfassen können, die jeweils mit zumindest einem der ersten Medienstecker 31, 32, 33 und einem der dritten Medienstecker verbunden sind. Dabei können auch mehrere der Anschlüsse 39 mit einem der ersten Medienstecker 31, 32, 33 oder einer der Anschlüsse 39 mit mehreren der Medienstecker 31, 32, 33 verbunden sein, wodurch Mediengemische bereitgestellt werden können.

Zwischen dem ersten Stromstecker 31 und dem dritten Stromstecker kann ein Trenntrafo od. dgl. zwischengeschaltet sein, um einen Stromfluß zwischen diesen Stromsteckern 31 zu unterbinden. Es kann auch eine Steuerung für ein im wesentlichen unterbrechungsfreies Umschalten einer Versorgung der Anschlüsse 39 über die ersten Medienstecker 31, 32, 33 zu einer Versorgung der Anschlüsse 39 über die dritten Medienstecker vorgesehen sein.

In Fig. 4 ist eine Ausführungsform der Verriegelung 6 dargestellt. Die Verriegelung 6 kann einen Griff 61, einen Schnappverschluß 62, eine Verschlußplatte 63, einen Schwenkstift 64 und ein Druckstück 65 umfassen. Bei der gezeigten Ausführungsform ist für die erste Seite 11 und für die zweite Seite 12 eine Verriegelung 6 vorgesehen.

In den Fig. 5 und 6 ist das zweite Verbindungsstück 2 dargestellt. Dieses weist einen Griff 24 und Eingänge 22 auf. Ein Trennblech 23 kann analog zum ersten Verbindungsstück 1 die Stromkammer 51 von der Gaskammer 52 trennen. Im Bereich der Gaskammer 52 können Belüftungsöffnungen 53 vorgesehen sein.

Wie aus Fig. 6 ersichtlich ist, können die zweiten Medienstecker 41, 42, 43 beweglich in dem zweiten Verbindungsstück 2 gelagert sein, wobei bei anderen Ausführungsformen einer, mehrere oder alle der zweiten Medienstecker 41, 42, 43 beweglich in dem zweiten Verbindungsstück 2 gelagert sein können. Anstelle der zweiten Medienstecker 41, 42, 43 können auch einer, mehrere oder alle der ersten Medienstecker 31, 32, 33 beweglich in dem ersten Verbindungsstück 1 gelagert sein. Dadurch wird eine Relativbewegung der Medienstecker 31, 32, 33, 41, 42, 43 ermöglicht, wodurch eine leichtere Betätigung der Medienstecker 31, 32, 33, 41, 42, 43 erreicht werden kann.

Der zweite Stromstecker 41 weist einen Schwenkteil 411, Bananenstecker 412 und einen Schwenkstift 413 auf. Anstelle der Bananenstecker 412 können auch andere Ausgestaltungen eines Stromsteckers vorgesehen sein.

Die zweiten Stecker für ein Fluid 42, 43 können jeweils eine Druckfeder 421, einen Druckstift 422, ein Schwenkteil 423 und ein Spannelement 424 umfassen.

Mit dem zweite Kontaktstift 44 kann analog dem ersten ein Schaltstift 441 betätigbar sein, welcher mit einem an einer Halteplatte 443 befestigten Schaltelement 442 verbunden ist.

Das erste Verbindungsstück 1 kann mit einem Gerät eine Einheit ausbildend verbunden sein, wodurch ein besonders einfacher Transport des Geräts mit dem ersten Verbindungsstück 1 ermöglicht wird.

## Patentansprüche

1. Medienversorgungssystem für medizinische Geräte mit einem ersten Verbindungsstück (1), welches zumindest eine erste Seite (11) mit wenigstens zwei ersten Mediensteckern (31, 32, 33) aufweist, wobei wenigstens ein zweites Verbindungsstück (2) vorgesehen ist, welches den ersten Mediensteckern (31, 32, 33) gegengleiche zweite Medienstecker (41, 42, 43) aufweist, wobei die Verbindungen der ersten Medienstecker (31, 32, 33) mit den korrespondierenden zweiten Mediensteckern (41, 42, 43) mittels einer Bewegung des zweiten Verbindungsstücks (2) gemeinsam herstellbar und/oder lösbar sind, **dadurch gekennzeichnet, dass** zumindest einer der ersten Medienstecker (31, 32, 33) ein erster Stromstecker (31) und zumindest einer der ersten Medienstecker (31, 32, 33) ein erster Stecker für ein Fluid (32, 33), insbesondere für ein Gas, ist, daß das zweite Verbindungsstück (2) um eine Schwenkachse (13) gegenüber dem ersten Verbindungsstück (1) verschwenkbar ist, und dass der Abstand des ersten Stromsteckers (31) von der Schwenkachse (13) größer als der Abstand des zumindest einen ersten Steckers für ein Fluid (32, 33) von der Schwenkachse (13) ist.

2. Medienversorgungssystem nach Anspruch 1, **dadurch gekennzeichnet, dass** die Schwenkachse (13) im Wesentlichen normal zur Längsachse der ersten Medienstecker (31, 32, 33) angeordnet ist.

3. Medienversorgungssystem nach Anspruch 1 oder 2, **dadurch gekennzeichnet, daß** das zweite Verbindungsstück (2) an einem der Schwenkachse (13) gegenüberliegenden Ende (21) mittels einem Verriegelungselement (6) lösbar mit dem ersten Verbindungsstück (1) verbindbar ist.

4. Medienversorgungssystem nach Anspruch 1, 2 oder 3, **dadurch gekennzeichnet, daß** das zweite Verbindungsstück (2) mit dem ersten Verbindungsstück (1) lösbar verbindbar ist, wobei die Schwenkachse (13) durch die Verbindung ausbildbar ist.

5. Medienversorgungssystem nach einem der Ansprüche 1 bis 4, **dadurch gekennzeichnet, daß** wenigstens einer der ersten Medienstecker (31, 32, 33) beweglich in dem ersten Verbindungsstück (1) und/oder wenigstens einer der zweiten Medienstecker (41, 42, 43) beweglich in dem zweiten Verbindungsstück (2) gelagert ist.

6. Medienversorgungssystem nach einem der Ansprüche 1 bis 5, **dadurch gekennzeichnet, daß** ein erster Kontaktstift (34) zum Schließen eines Stromkreises beweglich an der ersten Seite (11) des ersten Verbindungsstücks (1) angeordnet ist.

7. Medienversorgungssystem nach einem der Ansprüche 1 bis 6, **dadurch gekennzeichnet, daß** ein zweiter Kontaktstift (44) zum Schließen eines Stromkreises beweglich an dem zweiten Verbindungsstück (2) angeordnet ist.

8. Medienversorgungssystem nach einem der Ansprüche 2 bis 7, **dadurch gekennzeichnet, daß** der Stromstecker (31, 41) in einer Stromkammer (51) und der Stecker für ein Fluid (32, 33, 42, 43) in einer von der Stromkanuner (51) getrennten Gaskammer (52) angeordnet sind.

9. Medienversorgungssystem nach Anspruch 8, **dadurch gekennzeichnet, daß** die Gaskammer (52) Belüftungsöffnungen (53) aufweist.

10. Medienversorgungssystem nach einem der Ansprüche 1 bis 9, **dadurch gekennzeichnet, daß** das erste Verbindungsstück (1) wenigstens zwei erste Stecker für ein Fluid (32, 33) aufweist, wobei diese ersten Stecker (32, 33) identisch ausgebildet sind.

11. Medienversorgungssystem nach einem der Ansprüche 1 bis 10, **dadurch gekennzeichnet, daß** an dem ersten Verbindungsstück (1) wenigstens eine zweite Seite (12) mit dritten Mediensteckern für die Aufnahme eines weiteren zweiten Verbindungsstückes (2) ausgebildet ist.

12. Medienversorgungssystem nach Anspruch 11, **dadurch gekennzeichnet, daß** an dem ersten Verbindungsstück (1) Anschlüsse (39) vorgesehen sind, die jeweils mit zumindest einem der ersten Medienstecker (31, 32, 33) und einem der dritten Medienstecker verbunden sind.

13. Medienversorgungssystem nach Anspruch 11 oder 12, **dadurch gekennzeichnet, daß** den ersten Steckern für Fluide (32, 33) und den dritten Steckern für Fluide jeweils ein Rückschlagventil (35) zugeordnet ist.

14. Medienversorgungssystem nach Anspruch 11, 12 oder 13, **dadurch gekennzeichnet, daß** zwischen dem ersten Stromstecker (31) und dem dritten Stromstecker ein Trenntrafo od. dgl. zwischengeschaltet ist.

15. Medienversorgungssystem nach einem der Ansprüche 11 bis 14, **dadurch gekennzeichnet, daß** eine Steuerung für ein im wesentlichen unterbrechungsfreies Umschalten einer Versorgung der Anschlüsse (39) über die ersten Medienstecker (31, 32, 33) zu einer Versorgung der Anschlüsse (39) über die dritten Medienstecker vorgesehen ist.

## Claims

1. A media supply system for medical appliances, comprising a first connection element (1) which comprises at least a first side (11) with at least two first media plugs (31, 32, 33), with at least one second connection element (2) being provided which comprises second media plugs (41, 42, 43) which are diametrically opposite to the first media plugs (31, 32, 33), with the connections of the first media plugs (31, 32, 33) being producible with and/or detachable from the corresponding second media plugs (41, 42, 43) by means of a movement of the second connection element (2), **characterized in that** at least one of the first media plugs (31, 32, 33) is a first power plug (31) and at least one of the first media plugs (31, 32, 33) is a first plug for a fluid (32, 33), especially for a gas, that the second connection element (2) can be swiveled about a swiveling axis (13) relative to the first connection element (1), and that the distance of the first power plug (31) from the swiveling axis (13) is larger than the distance of the at least one first plug for a fluid (32, 33) from the swiveling axis (13).

2. A media supply system according to claim 1, **characterized in that** the swiveling axis (13) is arranged substantially normal to the longitudinal axis of the first media plugs (31, 32, 33).

3. A media supply system according to claim 1 or 2, **characterized in that** the second connection element (2) can be connected in a detachable manner with the first connection element (1) by means of a locking element (6) at an end (21) opposite of the swiveling axis (13).

4. A media supply system according to claim 1, 2 or 3, **characterized in that** the second connection element (2) can be detachably connected with the first connection element (1), with the swiveling axis (13) being formed by the connection.

5. A media supply system according to one of the claims 1 to 4, **characterized in that** at least one of the first media plugs (31, 32, 33) are movably held in the first connection element (1) and/or at least one of the second media plugs (41, 42, 43) is movably held in the second connection element (2).

6. A media supply system according to one of the claims 1 to 5, **characterized in that** a first contact pin (34) for closing a circuit is movably arranged on a first side (11) of the first connection element (1).

7. A media supply system according to one of the claims 1 to 6, **characterized in that** a second contact pin (44) for closing a circuit is movably arranged on the second connection element (2).

8. A media supply system according to one of the claims 2 to 7, **characterized in that** the power plug (31, 41) is arranged in a power chamber (51) and the plug for a fluid (32, 33, 42, 43) is arranged in a gas chamber (52) separate from the power chamber (51).

9. A media supply system according to claim 8, **characterized in that** the gas chamber (52) comprises ventilation openings (53).

10. A media supply system according to one of the claims 1 to 9, **characterized in that** the first connection element (1) comprises at least two first plugs for a fluid (32, 33), with said first plugs (32, 33) being arranged identically.

11. A media supply system according to one of the claims 1 to 10, **characterized in that** at least one second side (12) with third media plugs for receiving a further second connection element (2) is arranged on the first connection element (1).

12. A media supply system according to claim 11, **characterized in that** connections (39) are provided on the first connection element (1), which connections are each connected with at least one of the first media plugs (31, 32, 33) and one of the third media plugs.

13. A media supply system according to claim 11 or 12, **characterized in that** a non-return valve (35) is each associated with the first plugs for fluids (32, 33) and the third plugs for fluids.

14. A media supply system according to claim 11, 12 or 13, **characterized in that** an isolation transformer or the like is interposed between the first power plug (31) and the third power plug.

15. A media supply system according to one of the claims 11 to 14, **characterized in that** a control unit is provided for a substantially interruption-free changeover of a supply of the connections (39) via the first media plugs (31, 32, 33) to a supply of the connections (39) via the third media plugs.

## Revendications

1. Système d'alimentation en fluides pour des appareils médicaux, avec un premier élément de raccordement (1) comportant au moins un premier côté (11) avec au moins deux premiers connecteurs de fluides (31, 32, 33), dans lequel il est prévu au moins un deuxième élément de raccordement (2) qui possède des deuxièmes connecteurs de fluides (41, 42, 43) symétriques des premiers connecteurs de fluides (31, 32, 33), les assemblages des premiers connecteurs de fluides (31, 32, 33) avec les deuxièmes connecteurs de fluides (41, 42, 43) correspondants pouvant être établis et/ou défaits ensemble au moyen d'un mouvement du deuxième élément de raccordement (2), **caractérisé en ce que en ce que** l'un au moins des premiers connecteurs de fluides (31, 32, 33) est un premier connecteur électrique (31) et au moins un des premiers connecteurs de fluides (31, 32, 33) est un premier raccord pour un fluide (32, 33), en particulier pour un gaz, **en ce que** le deuxième élément de raccordement (2) peut pivoter autour d'un axe de pivotement (13) par rapport au premier élément de raccordement (1) et **en ce que** la distance entre le premier connecteur électrique (31) et l'axe de pivotement (13) est plus grande que la distance entre le premier connecteur au nombre d'un au moins pour un fluide (32, 33) et l'axe de pivotement (13).

2. Système d'alimentation en fluides selon la revendication 1, **caractérisé en ce que** l'axe de pivotement (13) est sensiblement perpendiculaire à l'axe longitudinal des premiers connecteurs de fluides (31, 32, 33).

3. Système d'alimentation en fluides selon la revendication 1 ou 2, **caractérisé en ce que** le deuxième élément de raccordement (2) peut être assemblé au premier élément de raccordement (1) de manière amovible, à une extrémité (21) opposée à l'axe de pivotement (13), au moyen d'un élément de verrouillage (6).

4. Système d'alimentation en fluides selon la revendication 1, 2 ou 3, **caractérisé en ce que** le deuxième élément de raccordement (2) peut être assemblé de manière amovible avec le premier élément de raccordement (1), l'axe de pivotement (13) pouvant être formé par l'assemblage.

5. Système d'alimentation en fluides selon l'une des revendications 1 à 4, **caractérisé en ce que** l'un au moins des premiers connecteurs de fluides (31, 32, 33) est supporté de manière mobile dans le premier élément de raccordement (1) et/ou au moins un des deuxièmes connecteurs de fluides (41, 42, 43) dans le deuxième élément de raccordement (2)

6. Système d'alimentation en fluides selon l'une des revendications 1 à 5, **caractérisé en ce qu'**une première goupille de contact (34) destinée à fermer un circuit électrique est disposée de manière mobile sur le premier côté (11) du premier élément de raccordement (1).

7. Système d'alimentation en fluides selon l'une des revendications 1 à 6, **caractérisé en ce qu'**une deuxième goupille de contact (44) destinée à fermer un circuit électrique est disposée de manière mobile sur le deuxième élément de raccordement (2).

8. Système d'alimentation en fluides selon l'une des revendications 2 à 7, **caractérisé en ce que** le connecteur électrique (31, 41) est disposé dans un compartiment électrique (51) et le connecteur pour un fluide (32, 33, 42, 43) dans un compartiment de gaz (52) séparé du compartiment électrique (51).

9. Système d'alimentation en fluides selon la revendication 8, **caractérisé en ce que** le compartiment de gaz (52) présente des ouvertures d'aération (53).

10. Système d'alimentation en fluides selon l'une des revendications 1 à 9, **caractérisé en ce que** le premier élément de raccordement (1) présente au moins deux premiers connecteurs pour un fluide (32, 33), ces premiers connecteurs (32, 33) étant de forme identique.

11. Système d'alimentation en fluides selon l'une des revendications 1 à 10, **caractérisé en ce que** le premier élément de raccordement (1) comporte au moins un deuxième côté (12) avec des troisièmes connecteurs de fluides pour recevoir un autre deuxième élément de raccordement (2).

12. Système d'alimentation en fluides selon la revendication 11, **caractérisé en ce qu'**il est prévu sur le premier élément de raccordement (1) des raccords (39) qui sont assemblés chacun à au moins un des premiers connecteurs de fluides (31, 32, 33) et à un des troisièmes connecteurs de fluides.

13. Système d'alimentation en fluides selon la revendication 11 ou 12, **caractérisé en ce que** les premiers connecteurs de fluides (32, 33) et les troisièmes connecteurs de fluides sont associés chacun à une soupape anti-retour (35).

14. Système d'alimentation en fluides selon la revendication 11, 12 ou 13, **caractérisé en ce qu'**un transformateur sectionneur ou similaire est intercalé entre le premier connecteur électrique (31) et le troisième connecteur électrique.

15. Système d'alimentation en fluides selon l'une des revendications 11 à 14, **caractérisé en ce qu'**il est prévu une commande pour une commutation sensiblement sans interruption d'une alimentation des raccords (39) par les premiers connecteurs de fluides (31, 32, 33)) et une alimentation des raccords (39) par les troisièmes connecteurs de fluides.
